# EUROPÄISCHE PATENTSCHRIFT

(11) **EP 1 997 421 B1**
(45) Veröffentlichungstag und Bekanntmachung des Hinweises auf die Patenterteilung: **11.07.2012**
(21) Anmeldenummer: 08009839.5
(22) Anmeldetag: 29.05.2008
(51) Int. Cl.: A61B 1/00, A61B 1/05

(54) **Videoendoskop**
Video endoscope
Endoscope vidéo

(30) Priorität: 31.05.2007 DE 102007026234
(43) Veröffentlichungstag der Anmeldung: 03.12.2008
(73) Patentinhaber: Karl Storz GmbH & Co. KG, 78532 Tuttlingen (DE)
(72) Erfinder: Irion, Klaus M., Dr.-Ing., 78576 Emmingen-Liptingen (DE); Graf, Christian, 78576 Emmingen-Liptingen (DE); Schwarz, Peter, 78532 Tuttlingen (DE)
(74) Vertreter: Weller, Wolfgang

(56) Entgegenhaltungen:
- EP-A- 1 201 179
- EP-A- 1 721 568
- DE-B3-102004 023 866
- JP-A- 6 254 049
- US-A1- 2002 193 664

## Beschreibung

Die Erfindung betrifft ein Videoendoskop mit einem Arbeitsteil, wobei das Arbeitsteil einen Schaft aufweist, an dessen distalem Ende ein Objektiv und ein Bildsensor angeordnet sind, und wobei das Arbeitsteil ferner ein elektrisches Übertragungssystem zum Übertragen der Bildinformation und zumindest einen Lichtleiter zum Übertragen von Licht von proximal nach distal aufweist, und mit einem Bedienteil, wobei ein proximales Ende des Arbeitsteils mit einem distalen Ende des Bedienteils über eine Schnittstelle lösbar verbindbar ist, wobei am Arbeitsteil ein Schlauch angebracht ist, mit dem das Bedienteil umhüllbar ist.

Ein derartiges Videoendoskop ist aus der EP-A-1 721 568 bekannt.

Videoendoskope werden beispielsweise bei minimal-invasiven Eingriffen in der Arthroskopie, in der Bauch- und Brustkorbspiegelung, bei Leistenbrüchen sowie bei Gelenk- und Wirbelsäulenoperationen eingesetzt. Dabei unterstützen die Endoskope den Operateur bei der Durchführung der Operation, indem sie einen möglichst großen Sichtbereich im Operationsgebiet einsehbar machen.

Ein erster Bestandteil derartiger Endoskope ist ein Bildgebungssystem. Das Bildgebungssystem dient zum Empfangen von Beobachtungslicht aus dem Beobachtungsraum bzw. Operationsraum und zur Übertragung von Bildinformationen von distal nach proximal.

Das Bildgebungssystem kann herkömmlich aus einem optischen Bildübertragungssystem bestehen, das ein Objektiv im distalen Ende des Schaftes, ein sich daran proximal anschließendes Linsensystem, beispielsweise in Form eines Stablinsen-Systems, oder ein geordnetes Faserbündel und ein proximales Okular aufweist, durch das mit dem Auge beobachtet werden kann, oder an das eine Kamera anschließbar ist.

Ein elektronisches Bildgebungssystem weist im distalen Ende des Schafts eine Abbildungsoptik und einen Bildaufnahme-Chip auf, der die Lichtsignale in elektrische Signale umwandelt, die über elektrische Leitungen nach proximal übertragen werden. Am proximalen Ende ist ein Kameramodul vorhanden, das meist zusätzlich als Bedienteil ausgebildet ist, und das die elektronische Bildinformation zu einer Bildverarbeitungseinheit weiterleitet.

Ein zweiter Bestandteil derartiger Endoskope ist ein Beleuchtungssystem. Das Beleuchtungssystem dient zur Übertragung von Licht von proximal nach distal, um den Beobachtungsraum bzw. Untersuchungsraum mit Licht anzuleuchten.

Nach einem erfolgten chirurgischen Eingriff muss ein derartiges Videoendoskop gereinigt und durch einen Autoklaviervorgang sterilisiert werden. Bei praktischen Anwendungen derartiger Videoendoskope hat sich herausgestellt, dass bei Sterilisierungs- bzw. Autoklaviervorgängen, die in dem Temperaturbereich von 130 bis 140°C durchgeführt werden, insbesondere optische und elektronische Komponenten der Videoendoskope beeinträchtigt werden können.

Die bei den Autoklaviervorgängen stattfindenden Temperaturschwankungen verursachen erhebliche mechanische Dehnungsspannungen, die zum Bruch von Verbindungsstellen bzw. an einer Linse führen können. Ferner können dadurch auch die sehr empfindlichen elektronischen Teile des Videoendoskops, z.B. Bildsensoren mit Farbfilter, beeinträchtigt werden. Somit verkürzen die obengenannten Vorgänge die Lebensdauer des Endoskops. Darüber hinaus sind die Sterilisierungs- bzw. Autoklaviervorgänge zeit- und kostenintensiv.

Demnach wird nach Lösungen gesucht, bei denen auf die aufwendigen Sterilisierungs- bzw. Autoklaviervorgänge verzichtet werden kann.

Ein Videoendoskop ist z.B. aus der JP 06254049 A bekannt.

Das Bereitstellen eines wegwerfbaren Arbeitsteils löst zwar das Problem des Sterilisierens des Videoendoskops teilweise dadurch, dass ein vorab sterilisiertes Arbeitsteil als einmal verwendbares Bauteil mit einem Bedienteil verbunden werden kann, es besteht jedoch noch immer das Problem, dass das Bedienteil durch im Operationssaal vorhandene Fluide und Flüssigkeiten verschmutzt und kontaminiert werden kann, was z.B. zu Störungen in dem Bedienteil führen kann. Ferner muss das Bedienteil noch immer sterilisiert werden, wenn auch dies nicht mehr zu dem gleichen hohen Grad erfolgen muss, wie wenn das Bedienteil fest mit dem Arbeitsteil verbunden ist, das direkt in den Körper eines Patienten eingeführt wird. Jedoch auch weniger belastende Sterilisierungsverfahren wie z.B. das Einlegen des Bedienteils in eine Desinfektionsflüssigkeit können langfristig zu Störungen bis zum Ausfall des Bedienteils führen.

Bei der eingangs genannten EP-A-1 721 568 ist vorgesehen am proximalen Ende des Arbeitsteils,und zwar von der Schnittstelle mit dem Bedienteil nach proximal vorstehend, einen Schlauch vorzusehen. Der Schlauch bzw. das relativ kurze vorstehende Schlauchstück ist so ausgebildet, dass es über das Bedienteil geschoben werden kann.

Das distale Ende des Bedienteiles muss durch das Schlauchstück soweit hindurchgeschoben werden, bis es die Schnittstelle des Arbeitsteiles erreicht, denn erst dann sind die entsprechenden elektrischen licht- und bildleitenden Verbindungen hergestellt.

Ist der Schlauch als ein relativ steifes Rohrstück ausgebildet, kann das Einführen der Bedienteiles relativ einfach durchgeführt werden. Am Schlauchstück ist an der Stelle, an der die Bedienknöpfe des Bedienteiles zum Liegen kommen, eine leichte Erhebung vorhanden.

Ist der Schlauch aus einem schlaffen oder folienartigen Material ausgebildet, muss er mit relativ großer Aufmerksamkeit über das Bedienteil geschoben werden. Durch den Schlauch ist die eigentliche Kupplungsstelle zwischen Arbeitsteil und Bedienteil abgedeckt, so dass ein gezieltes und lagegerechtes Zusammenstecken von Arbeitsteil und Bedienteil erschwert ist.

Es ist Aufgabe der Erfindung, ein Videoendoskop der eingangs genannten Art dahingehend weiterzuentwickeln, dass auf einfache Weise das Bedienteil vor Kontaminationen im Operationssaal geschützt wird, wobei dies durch einfache und kostengünstige Bauteile durchgeführt wird.

Erfindungsgemäß wird die Aufgabe dadurch gelöst, dass der Schlauch als steriler zusammengeraffter Schlauch ausgebildet ist, und dass das Arbeitsteil als Einweginstrument ausgebildet ist.

Diese Maßnahme hat den Vorteil, dass das Bedienteil, das mehrere sterilisationsempfindliche Elemente aufweist, durch den Schlauch vor Kontaminationen geschützt ist. Das Arbeitsteil wird mit dem nicht sterilisierten Bedienteil verbunden. Dann wird das Arbeitsteil von einer sterilen Hand des Operateurs ergriffen und mit der zweiten Hand wird der sterile Schlauch, der an dem Arbeitsteil angeordnet ist, über das nicht sterilisierte Bedienteil gezogen, so dass der Schlauch das Bedienteil umhüllt. Nach dem Einsatz wird das Arbeitsteil von dem Bedienteil getrennt und das Arbeitsteil mit dem Schlauch entsorgt. Dieser Vorgang der Umhüllung kann auch unter Einsatz einer Andockvorrichtung zwangsgeführt erfolgen.

In einer weiteren Ausgestaltung der Erfindung ist die Schnittstelle derart ausgebildet, dass die Bildinformation von dem Bildsensor an das Bedienteil übertragbar ist.

Diese Maßnahme hat den Vorteil, dass durch eine derartige Ausgestaltung der Schnittstelle die Kopplung der für die Bildübertragung verantwortlichen Elemente gewährleistet wird. Die Bildinformation wird von dem Sensor mittels des elektrischen Übertragungssystems über die Schnittstelle an das Bedienelement übertragen, so dass die Bildinformation nach einer Bildverarbeitung auf einem Monitor als Bild dargestellt werden kann. Dadurch kann der Operateur das endoskopische Operationsfeld auf dem Monitor beobachten.

In einer weiteren Ausgestaltung der Erfindung ist die Schnittstelle derart ausgebildet, dass Licht von dem Bedienteil an das Arbeitsteil übertragbar ist.

Diese Maßnahme hat den Vorteil, dass Licht vom Bedienteil an das distale Ende des Arbeitsteils geleitet wird, wodurch der Untersuchungsraum mit Licht angeleuchtet wird.

In einer weiteren Ausgestaltung der Erfindung weist das Arbeitsteil ein Außenteil auf, das als Schaft ausgebildet ist.

Diese Maßnahme hat den Vorteil, dass der Schaft eine Schutzfunktion erfüllt, da die sich in dem Schaft befindlichen Elemente, wie Sensor, Übertragungssystem usw. vor Beeinträchtigen bspw. durch angreifende Verbiegungskräfte geschützt werden. Dieser Schaft kann als Kunststoffschaft ausgebildet werden und als Spritzgussteil ausgeführt werden. Er ist somit extrem kostengünstig umsetzbar.

In einer weiteren Ausgestaltung der Erfindung ist in dem Schaft der zumindest eine Lichtleiter angeordnet.

Diese Maßnahme hat den Vorteil, dass der Schaft zusätzlich zu der Schutzfunktion der in dem Endoskop angeordneten empfindlichen Elemente ebenfalls den Lichtleiter schützt. Der Schaft kann aus einem lichtleitenden Kunststoff ausgebildet werden. Dies trägt zu einem einfachen und kostengünstigen Aufbau des Arbeitsteils bei.

In einer weiteren Ausgestaltung der Erfindung weist das Arbeitsteil ein Innenteil auf, in dem der Bildsensor und das elektrische Übertragungssystem angeordnet sind.

Diese Maßnahme hat den Vorteil, dass durch eine derartige Ausgestaltung des Arbeitsteils einer Beschädigung des Bildsensors und des Bildübertragungssystems vorgebeugt wird, da die Elemente von dem Innenteil umschlossen sind und somit geschützt werden.

In einer weiteren Ausgestaltung der Erfindung ist das Außenteil relativ zu dem Innenteil drehbar.

Diese Maßnahme hat den Vorteil, dass durch Drehbarkeit des Außenteils mit dem Objektiv gegenüber dem Bildsensor die sogenannte Bildaufrichtung gewährleistet wird.

Unter Bildaufrichtung versteht man eine bestimmte Horizontal-Ausrichtung des vom Kameramodul erzeugten Monitorbildes nach Verdrehen des Endoskops meist mit asymmetrischer Blickrichtung (z.B. 30°) um die Schaftachse. Manche Operateure wünschen eine gleichbleibende Horizontal-Ausrichtung des auf dem Monitor ersichtlichen Operationsbildes, wenn während einer Operation das Endoskop aus einer Ausgangsstellung verdreht wird. Dazu ist es an sich bekannt geworden, die Bildaufnahmeeinheit entsprechend nachzudrehen, um dadurch das Bild wieder aufzurichten.

In einer weiteren Ausgestaltung der Erfindung ist ein Mechanismus zum Drehen des Außenteils vorgesehen.

Diese Maßnahme hat den Vorteil, dass die Aufrichtung des Bilds durch Betätigen des Mechanismus einfach, sogar einhändig durch den Operateur während des chirurgischen Eingriffs durchgeführt werden kann.

Der Mechanismus kann manuell bspw. mittels eines Betätigungshebels oder motorisch angetrieben werden. Für den Antrieb der motorischen Rotation des Außenteils kann das Arbeitsteil bspw. einen Reibbereich bzw. eine Zahnung und das Bedienteil eine Antriebsrolle bzw. ein Zahnrad aufweisen. Sowohl der manuell als auch motorisch angetriebene Mechanismus sind konstruktiv einfach zu realisieren.

In einer weiteren Ausgestaltung der Erfindung ist der Bildsensor als CMOS-Sensor ausgebildet.

Diese Maßnahme hat den Vorteil, dass derartige Bildsensoren sich in der Praxis als besonders vorteilhaft hinsichtlich ihrer Auflösung herausgestellt haben. Die CMOS-Technologie weist gegenüber den CCD-Sensoren einen deutlich geringeren Energieverbrauch auf und erlaubt bei einem größeren Produktionsvolumen eine günstige Produktion, da sie ohne Umrüstung auf den für hohe Stückzahlen ausgelegten Fertigungsstraßen gefertigt werden können und so einen geringen Fertigungsaufwand pro Chip verursachen. Somit lässt sich das wegwerfbare Arbeitsteil besonders kostengünstig herstellen.

In einer weiteren Ausgestaltung der Erfindung, die alternativ zu der zuvor genannten Ausgestaltung verwendet werden kann, ist der Bildsensor als CCD-Sensor ausgebildet.

Diese Maßnahme hat den Vorteil, dass die CCD-Sensoren üblicherweise lichtempfindlicher als übliche CMOS-Sensoren sind.

In einer weiteren Ausgestaltung der Erfindung ist das elektrische Übertragungssystem als zumindest eine durchgehende Platine ausgebildet.

Diese Maßnahme hat den Vorteil, dass eine durchgehende Platine eine konstruktiv einfache und gleichzeitig kostengünstige Ausgestaltung darstellt. Ferner werden bei einer derartigen Ausgestaltung des Übertragungssystems keine zusätzlichen Kabelanschlüsse und Kontaktierungen notwendig. Dies trägt zu einem einfachen und montagefreundlichen Aufbau des Arbeitsteils bei.

In einer weiteren Ausgestaltung der Erfindung ist ein proximales Ende der Platine als Stecker ausgebildet.

Diese Maßnahme hat den Vorteil, dass durch den Stecker die Bildinformation von dem Bildsensor, der in dem Arbeitsteil angeordnet ist, an das Bedienteil übertragen wird. Ferner hat eine derartige Ausgestaltung des Arbeitsteils den Vorteil, dass die kostengünstige Platine multifunktionell ausgebildet ist, so dass kein zusätzliches Bauelement verwendet werden muss. Dies trägt zu einem geringeren Montageaufwand bei.

In einer weiteren Ausgestaltung der Erfindung sind der Bildsensor und die Platine von einem zylinderförmigen Element umschlossen.

Diese Maßnahme hat den Vorteil, dass durch das zylinderförmige Element die gesamte Anordnung bestehend aus dem Bildsensor und der Platine stabilisiert bzw. verstärkt wird. Andererseits dient das zylinderförmige Element auch vorteilhafterweise der Isolation der Leiterdrähte.

In einer weiteren Ausgestaltung der Erfindung weist das Arbeitsteil zumindest einen Kanal auf.

Diese Maßnahme hat den Vorteil, dass der zumindest eine Kanal, z.B. zur Spülung und Säuberung des Objektivs oder zum Zuführen eines medizinischen Instruments dienen kann. Der Kanal mündet vorzugsweise seitlich vom Schaft abgehend, und zwar vor dem Kopplungsbereich mit dem Bedienteil.

In einer weiteren Ausgestaltung der Erfindung weist das proximale Ende des Arbeitsteils einen größeren Durchmesser auf als das distale Ende.

Diese Maßnahme hat den Vorteil, dass der distale Endbereich des Arbeitsteils durch eine derartige Ausgestaltung genügend Platz für das elektrische Steckersystem und die koaxial angeordnete Lichtkopplung aufweist.

In einer weiteren Ausgestaltung der Erfindung sind in dem Arbeitsteil LEDs angeordnet.

Diese Maßnahme hat den Vorteil, dass die LEDs, die über den Stecker elektrisch versorgt werden können, Licht in das Außenteil einkoppeln.

In einer weiteren Ausgestaltung der Erfindung sind in dem Innenteil des Arbeitsteils LEDs angeordnet.

Diese Maßnahme hat den Vorteil, dass durch die LEDs Licht radial in das Außenteil eingekoppelt werden kann, welches dort über Spiegelelemente nach distal umgelenkt wird.

In einer weiteren Ausgestaltung der Erfindung ist an dem proximalen Ende des Arbeitsteils ein Lichteintritt angeordnet.

Diese Maßnahme hat wiederum den Vorteil, dass Licht von dem Bedienteil über den Lichteintritt an das Arbeitsteil übertragen werden kann, so dass der Operationsbereich angeleuchtet wird.

In einer weiteren Ausgestaltung der Erfindung ist an dem distalen Ende des Bedienteils ein Lichtaustritt angeordnet.

Diese Maßnahme hat den Vorteil, dass durch den Lichtaustritt Licht von dem Bedienteil an das Arbeitsteil übertragen werden kann. Der Lichtaustritt des Bedienteils ist derart ausgebildet, dass er, nachdem das Bedienteil mit dem Arbeitsteil verbunden ist, mit dem Lichteintritt des Arbeitsteils in direktem Kontakt steht. Der Lichtaustritt weist entweder LEDs oder Lichtfasern auf.

In einer weiteren Ausgestaltung der Erfindung ist an dem distalen Ende des Bedienteils eine Buchse zur Aufnahme des Steckers angeordnet.

Diese Maßnahme hat den Vorteil, dass auf eine konstruktiv einfache Weise eine elektrische Kopplung zwischen dem Arbeitsteil und Bedienteil geschaffen wird.

In einer weiteren Ausgestaltung der Erfindung weist das Bedienteil Bedienelemente auf.

Diese Maßnahme hat den Vorteil, dass der Operateur die Kamera oder sonstige Schalter oder Ventile durch einfaches Betätigen der an dem Bedienteil angeordneten Bedienelemente bedienen kann.

In einer weiteren Ausgestaltung der Erfindung sind im distalen Bereich des Bedienteils umfänglich LEDs angeordnet, die den Lichteintritt des Arbeitsteils beleuchten.

Diese Maßnahme hat den Vorteil, dass umfänglich gleichmäßig verteilt das Licht eingekoppelt werden kann und dass dies auch bei einer vorhandenen Drehbarkeit in jeder Drehstellung gewährleistet ist.

In einer weiteren Ausgestaltung der Erfindung sind im distalen Bereich des Bedienteils umfänglich verteilt Lichtfasern angeordnet, die den Lichteintritt des Arbeitsteils beleuchten.

Diese Maßnahme hat denselben Vorteil wie zuvor erwähnt, wobei das Licht an den distalen Bereich des Bedienteils durch Lichtleitfasern geführt wird.

In einer weiteren Ausgestaltung der Erfindung sind im Bedienteil umfänglich verteilt LEDs angeordnet, die Licht radial in das Arbeitsteil einkoppeln, welches über Spiegelelemente nach distal umlenkbar ist.

Auch diese Lichteinkopplungsmöglichkeit erlaubt in jeder Drehstellung der Bauelemente untereinander eine effektive Lichteinkopplung.

Diese Art der Einkopplung kann auch in dem Arbeitsteil selbst vorhanden sein, wenn beispielsweise Arbeitsteil und Bedienteil nicht drehbar miteinander verkoppelt sind und das Arbeitsteil ein Innenteil und ein dazu drehbares Außenteil aufweist.

Weitere Merkmale und Vorteile ergeben sich aus der nachfolgenden Beschreibung und den beiliegenden Zeichnungen.

Es versteht sich, dass die vorstehend genannten und die nachstehend noch zu erläuternden Merkmale nicht nur in der angegebenen Kombination, sondern auch in anderen Kombinationen oder in Alleinstellung einsetzbar sind, ohne den Rahmen der vorliegenden Erfindung zu verlassen.

Die Erfindung wird nachfolgend anhand ausgewählter Ausführungsbeispiele in Zusammenhang mit den beiliegenden Zeichnungen näher beschrieben und erläutert. Zur besseren Übersichtlichkeit ist in den Figuren 2 sowie 8 bis 10 der erfindungsgemäße Schlauch nicht dargestellt. Es zeigen:
- Fig. 1: eine perspektivische Ansicht eines erfindungsgemäßen Videoendoskops, wobei ein Arbeitsteil und ein Bedienteil voneinander getrennt sind;
- Fig. 2: eine vergrößerte perspektivische Ansicht eines proximalen Endbereichs des Arbeitsteils;
- Fig. 3: eine vergrößerte teilweise geschnittene Ansicht eines distalen Endbereichs des Arbeitsteils;
- Fig. 4: eine vergrößerte perspektivische Ansicht des Bedienteils;
- Fig. 5: eine der Darstellung in Fig. 1 vergleichbare Darstellung, wobei das Bedienteil und das Arbeitsteil verbunden sind;
- Fig. 6: eine der Darstellung in der Fig. 1 vergleichbare Darstellung, wobei ein weiteres Ausführungsbeispiel des Arbeitsteils dargestellt ist;
- Fig. 7: eine der Darstellung in Fig. 5 vergleichbare Darstellung, wobei das Bedienteil mit dem sterilen Schlauch umhüllt ist;
- Fig. 8: einen Längsschnitt im Kopplungsbereich zwischen einem Arbeitsteil und einem Bedienteil mit unterschiedlichen Ausgestaltungen der Lichteinkopplung, in der oberen Hälfte über LEDs im Bedienteil auf Lichtleitfasern im Arbeitsteil, in der unteren Hälfte von Lichtleitfasern im Bedienteil auf Lichtleitfasern im Arbeitsteil;
- Fig. 9: einen proximalseitigen Längsschnitt einer weiteren Ausgestaltung eines Arbeitsteils mit einer darin integrierten Lichtquelle in Form von LEDs; und
- Fig. 10: einen Schnitt im Kopplungsbereich zwischen einem Arbeitsteil und einem Bedienteil, wobei Licht vom Bedienteil in radialer Richtung in das Arbeitsteil eingestrahlt wird, in dem es durch Spiegelelemente nach distal gelenkt wird.

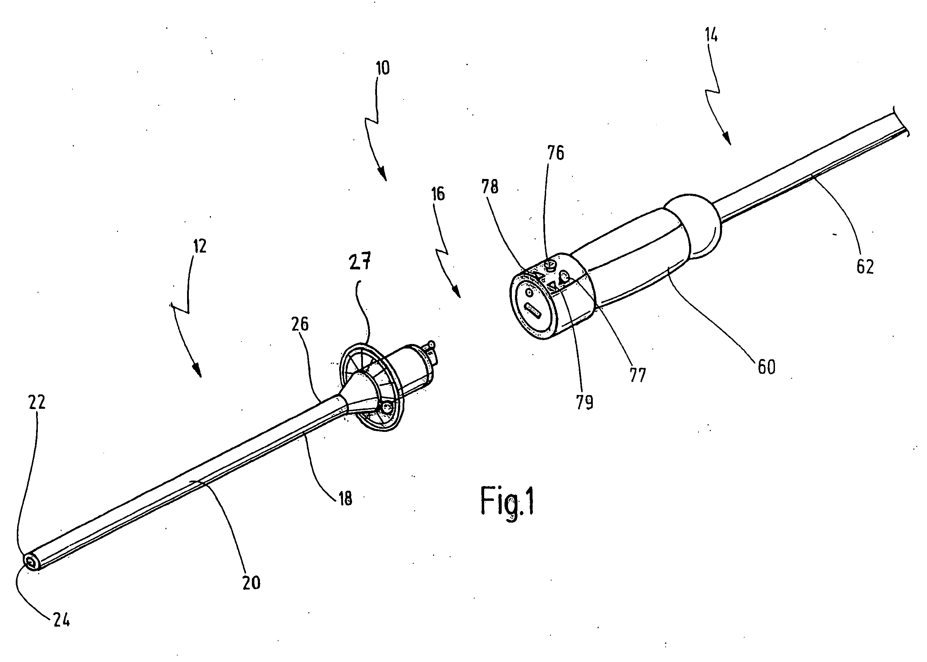

Ein in den Fig. 1 bis 7 dargestelltes Videoendoskop ist in seiner Gesamtheit mit der Bezugsziffer 10 versehen.

Das Videoendoskop 10 weist ein Arbeitsteil 12 und ein Bedienteil 14 auf, die über eine Schnittstelle 16 miteinander lösbar verbindbar sind, wie es noch später näher beschrieben wird.

Das Arbeitsteil 12 ist als Einweginstrument und das Bedienteil 14 ist als Mehrweginstrument ausgebildet.

Das Arbeitsteil 12 weist ein Außenteil 18 auf, das in diesem Ausführungsbeispiel als Schaft 20 ausgebildet ist. Der Schaft 20 weist zumindest einen Lichtleiter 26 auf, der Licht von proximal nach distal leitet. In diesem Ausführungsbeispiel ist der Schaft 20 aus einem lichtleitenden Kunststoff ausgebildet.

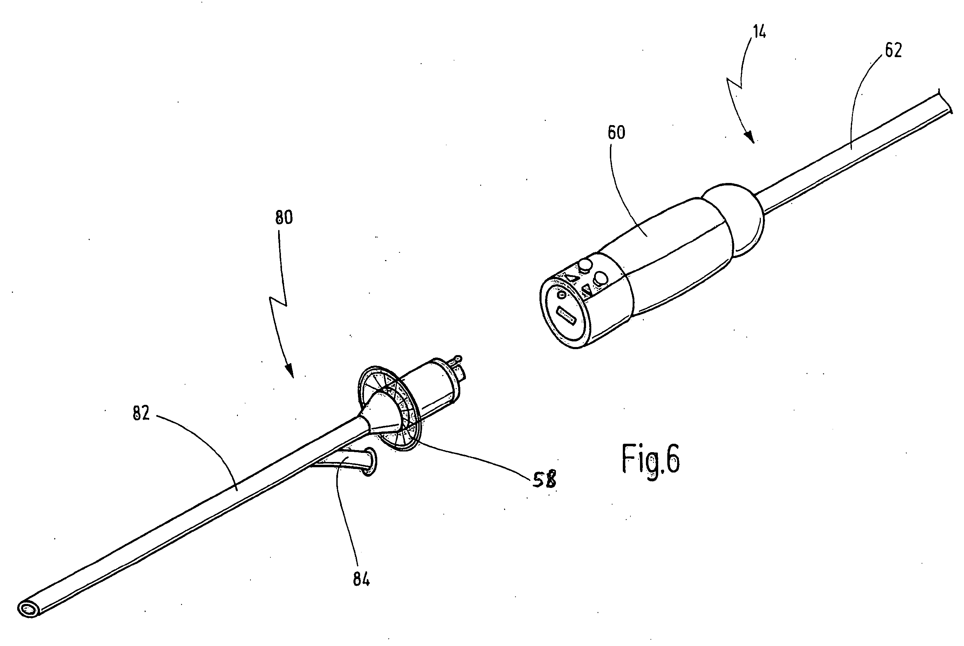

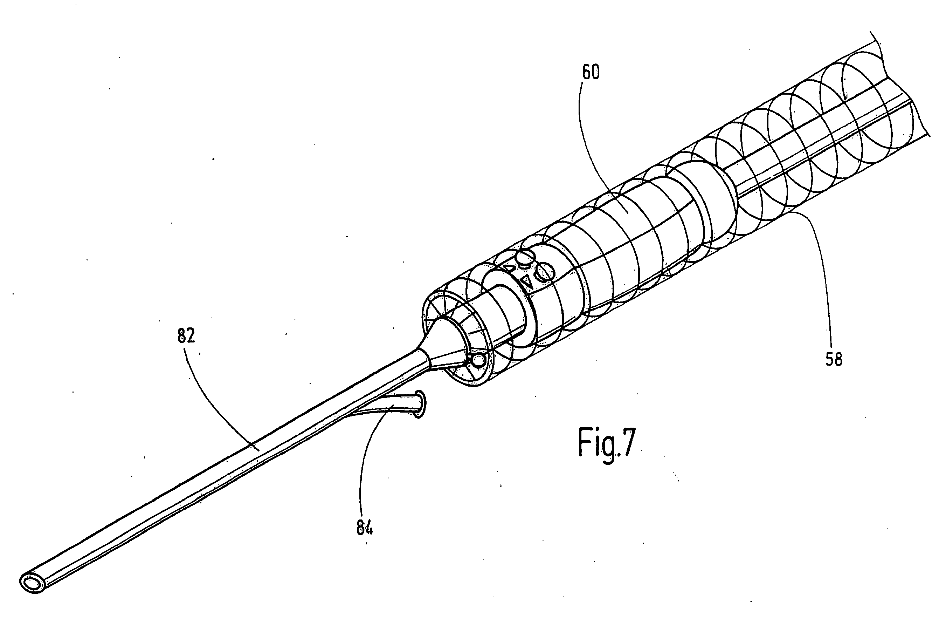

Am proximalen Ende des Arbeitsteils 12 ist ein zusammengeraffter Schlauch 27 angeordnet, dessen genaue Funktionsweise nachstehend mit Bezug auf Fig. 6 und 7 näher erläutert werden wird.

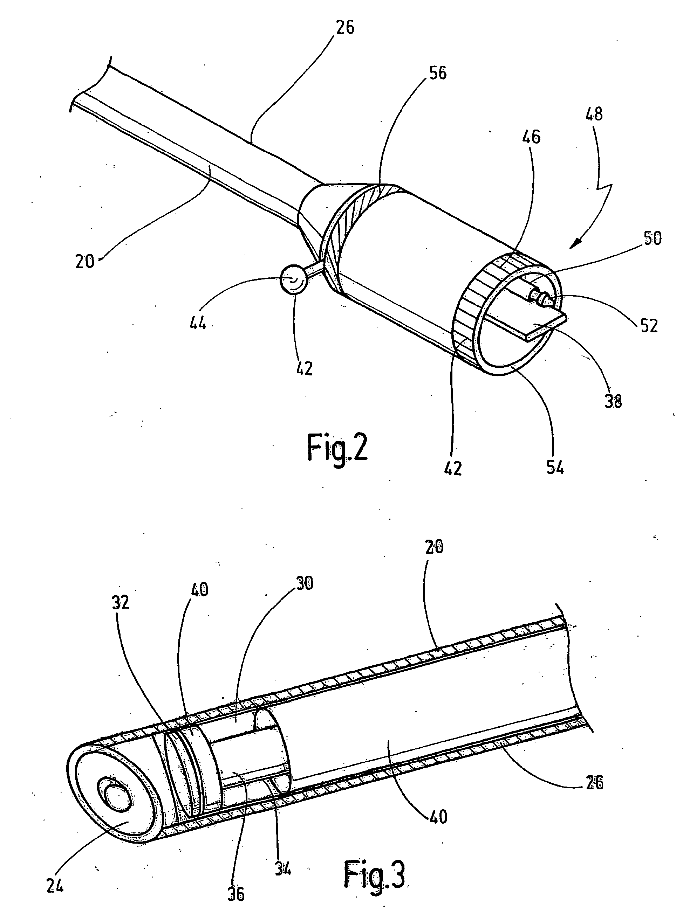

Der Schaft 20 ist an seinem distalen Ende 22 mit einem Objektiv 24 hermetisch abgeschlossen, wie es insbesondere aus der vergrößerten Darstellung in Fig. 3 ersichtlich ist.

Der aus einem lichtleitenden Kunststoff ausgebildete Schaft 20 wird in einem Spritzgussvorgang hergestellt. Dabei kann gleichzeitig ein Teil des Objektives 24 mit eingespritzt werden.

Das Arbeitsteil 12 weist ferner ein Innenteil 30 auf, das insbesondere aus der Darstellung in Fig. 3 ersichtlich ist.

In dem Innenteil 30 ist ein Bildsensor 32 aufgenommen, der in diesem Ausführungsbeispiel als CMOS-Sensor ausgebildet ist. Der Sensor 32 wandelt die Lichtsignale in elektrische Signale um.

Ferner ist in dem Innenteil 30 ein elektrisches Übertragungssystem 34 angeordnet, das die Bildsignale von dem Sensor 32 nach proximal überträgt. In diesem Ausführungsbeispiel ist das elektrische Übertragungssystem 34 als eine durchgehende Platine 36 ausgebildet.

Ein distales Ende der Platine 36 ist als ein Stecker 38 ausgebildet, der insbesondere aus der vergrößerten Darstellung in Fig. 2 ersichtlich ist. Die Funktion des Steckers 38 wird noch später näher beschrieben.

Der Bildsensor 32 und das als Platine 36 ausgebildete elektrische Übertragungssystem 34 sind von einem zylinderförmigen Element 40 umschlossen. Das aus einer Vergussmasse ausgebildete zylinderförmige Element 40 dient zur Stabilisierung des Bildsensors 32 und der Platine 36 und zur elektrischen Isolation.

Das Außenteil 18 ist relativ zu dem Innenteil 30 drehbar. Die Drehbarkeit des an dem distalen Ende des Schaftes 20 angeordneten Objektivs 24 gegenüber dem im Innenteil 30 angeordneten Bildsensor 32 ermöglicht die Bildaufrichtung.

Ferner ist ein Mechanismus 42 zum Drehen des Außenteils 18 vorgesehen. Der Mechanismus 42 kann manuell oder motorisch angetrieben werden.

Zum manuellen Antreiben des Mechanismus 42 ist ein Betätigungshebel 44 vorgesehen, der proximalseitig an dem Arbeitsteil 12 angeordnet ist. Durch manuelles Drehen des Betätigungshebels 44 wird das Außenteil 18 des Arbeitsteils 12 in Bezug auf das Innenteil 30 gedreht.

Zum motorischen Antreiben des Mechanismus 42 weist das Arbeitsteil 12 eine Zahnung bzw. einen Reibbereich 46 auf, die/der an dem proximalen Ende 48 des Arbeitsteils 12 angeordnet ist. Das Bedienteil 14 weist an seinem distalen Ende 64 dagegen ein Zahnrad bzw. ein Reibrad 72 auf, das nachdem die beiden Elemente 12 und 14 verbunden sind, mit der Zahnung bzw. dem Reibbereich 46 des Arbeitsteils 12 zusammenwirkt.

An dem proximalen Ende 48 des Arbeitsteils 12 ist ein Verriegelungselement 50 angeordnet, das zum Verriegeln des Arbeitsteils 12 an dem Bedienteil 14 dient. In diesem Ausführungsbeispiel ist das Verriegelungselement 50 als ein Bolzen 52 ausgebildet.

An dem proximalen Ende 48 des Arbeitsteils 12 ist ferner ein ringförmiger Lichteintritt 54 angeordnet, mittels dessen Licht von dem Bedienteil 14 an das Arbeitsteil 12 übertragen wird.

Der Bereich 56 gibt hierbei den Bereich wieder, an dem der hier nicht dargestellte Schlauch 27 mit dem Arbeitsteil 12 verbunden ist.

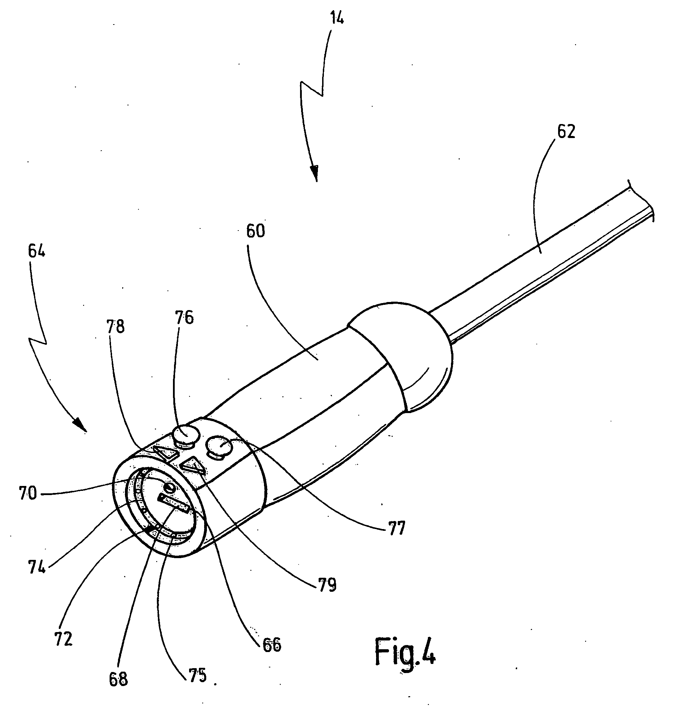

Das Bedienteil 14 ist als Handgriff 60 ausgebildet, wie das insbesondere aus Fig. 4 hervorgeht. Der Handgriff 60 ist mit einem Kabel 62 verbunden. In dem Bedienteil 14 sind sterilisationsempfindliche, elektronische Elemente, die hier nicht dargestellt sind, aufgenommen.

Das Bedienteil 14 weist an seinem distalen Ende 64 eine einen Schlitz 68 aufweisende Buchse 66 auf, in den der Stecker 38 der Platine 36 eingeführt wird. Dadurch wird eine elektrische Kopplung geschaffen, die die Übertragung der Bildinformation von dem Bildsensor 32 an das Bedienteil 14 ermöglicht. Die übertragene Bildinformation wird nach einer Bildverarbeitung für den Operateur als Bild auf einem Monitor ersichtlich.

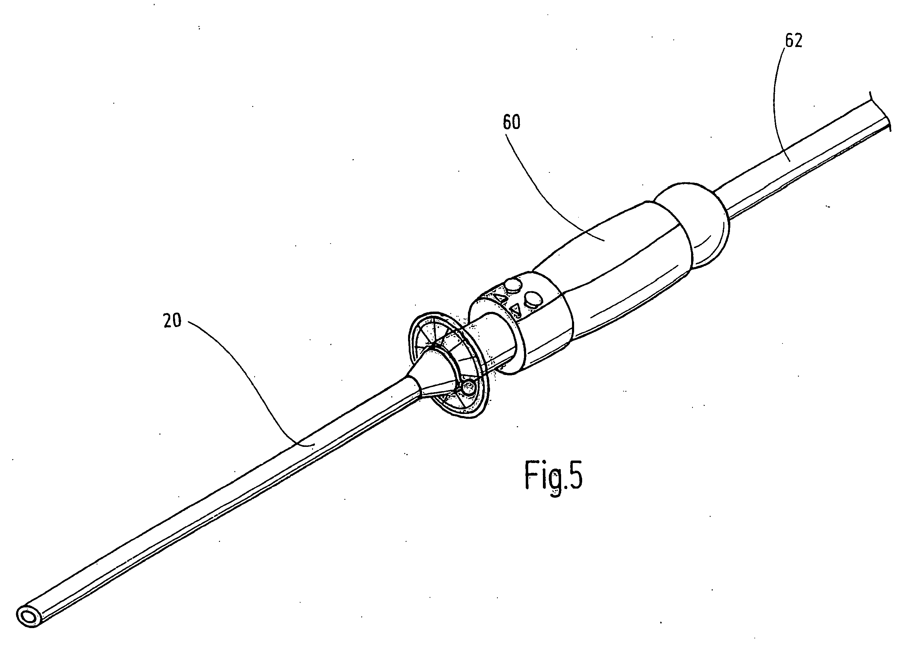

Ferner ist an dem distalen Ende 64 des Bedienteils 14 eine Ausnehmung 70 vorgesehen, in die das als Bolzen 52 ausgebildete Verriegelungselement 50 des Arbeitsteils 12 eingeführt werden kann, wodurch das Arbeitsteil 12 mit dem Bedienteil 14 verbunden wird. Solch eine Situation ist in Fig. 5 dargestellt.

An dem distalen Ende 64 des Bedienteils 14 ist ebenfalls ein Lichtaustritt 74 angeordnet, der in diesem Ausführungsbeispiel ringförmig ausgebildet ist. In diesem Ausführungsbeispiel sind an dem Lichtaustritt 72 LEDs 75 angeordnet. Der Lichtaustritt 72 des Bedienteils 14 ist derart angeordnet und ausgebildet, dass er nach Verbinden des Arbeitsteils 12 mit dem Bedienteil 14 mit dem Lichteintritt 54 des Arbeitsteils 12 in Kontakt steht. Dadurch wird Licht von dem Bedienteil 14 an das Arbeitsteil 12 übertragen.

An dem als Handgriff 60 ausgebildeten Bedienteil 14 sind Bedienelemente 76, 77, 78, 79 angeordnet, mittels derer die Kamera betätigt wird.

In Fig. 6 ist ein weiteres Ausführungsbeispiel für ein Arbeitsteil 80 dargestellt, das ebenfalls einen Schaft 82 aufweist. Das Arbeitsteil 80 weist die gleichen Elemente auf, wie das in Fig. 1 bis 5 dargestelltes Arbeitsteil 12. Die entsprechenden Elemente werden mit den gleichen Bezugsziffern wie bei dem Arbeitsteil 12 versehen.

Das in Fig. 6 dargestellte Arbeitsteil 80 unterscheidet sich von dem Arbeitsteil 12 darin, dass es einen seitlich austretenden Instrumentenkanal 84 aufweist, über den ein Instrument einführbar ist. An das Ende kann z.B. ein Schlauch angeschlossen werden. Der Instrumentenkanal 84 tritt vor dem Bereich aus, an dem die Bauteile zusammengesteckt werden.

Vor einem chirurgischen Eingriff wird das Arbeitsteil 80 mit dem sterilen Schlauch 58 aus einem hier nicht dargestellten sterilen Überzug entnommen und mit dem nicht sterilisierten Bedienteil 14 verbunden. Dann wird der an dem Arbeitsteil 80 fixierte sterile Schlauch 58 über das als Handgriff 60 ausgebildete Bedienteil 14 gezogen, so dass das nicht sterilisierte Bedienteil 14 mit dem sterilen Schlauch 58 umhüllt ist. Solch eine Situation ist in Fig. 7 dargestellt.

Dadurch wird sichergestellt, dass das sterile Arbeitsteil 80 nicht von dem nicht sterilisierten Bedienteil 14 kontaminiert wird. Nach einem Einsatz wird das Arbeitsteil 80 mit dem sterilen Schlauch 27 von dem Bedienteil 14 getrennt und entsorgt.

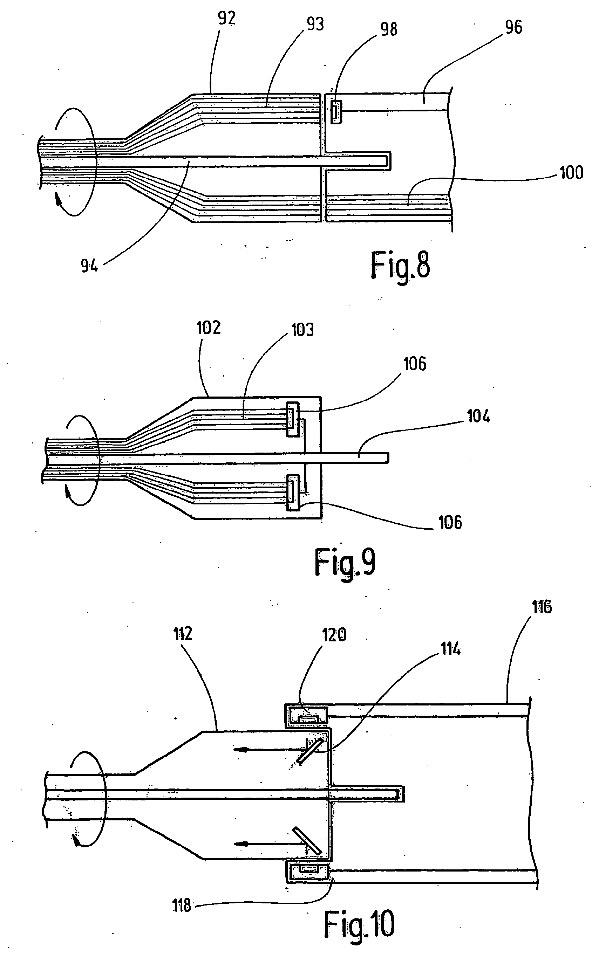

In Fig. 8 ist ein Arbeitsteil 92 dargestellt, das über einen Stecker 94 mit einem Bedienteil 96 gekoppelt ist.

Im Arbeitsteil 92 sind umfänglich verteilt Lichtleitfasern 93 angeordnet, die vom proximalen bis zum distalen Ende des Arbeitsteiles 92 reichen. In der oberen Hälfte von Fig. 8 ist eine Variante der Lichteinkopplung in das Arbeitsteil dargestellt, nämlich durch entsprechend umfänglich angeordnete LEDs 98. In der unteren Hälfte ist eine andere Möglichkeit dargestellt, nämlich durch entsprechend umfänglich verteilte Lichtleitfasern 100 im Bedienteil 96.

In Fig. 9 ist eine Variante dargestellt, bei der im Arbeitsteil 102 umfänglich verteilt Lichtleitfasern 103 angeordnet sind, an deren proximalen Ende LEDs 106 angeordnet sind. Die LEDs 106 sind über Leitungen elektrisch mit dem Stecker 104 verbunden und werden über diesen Stecker 104 mit Energie versorgt, wenn dieses Arbeitsteil 102 mit einem entsprechenden Bedienteil gekoppelt ist. Hier wird also das Licht im Arbeitsteil 102 selbst erzeugt, nämlich über die LEDs 106, das dann über die Lichtleitfasern 103 zum distalen Ende geführt wird. Die Energieeinspeisung erfolgt nach Kopplung mit dem Bedienteil. Somit ist auch hier das Arbeitsteil 102 als kostengünstiges Bauteil, also als Wegwerfteil realisierbar.

In Fig. 10 ist eine weitere Variante der Lichteinkopplung gezeigt. In diesem Ausführungsbeispiel sind im Bedienteil 116 in einem distal vorstehenden umfänglichen Flansch 118 LEDs 120 angeordnet. Diese LEDs 120 strahlen Licht nach radial ab.

Ist das Bedienteil 116 mit einem Arbeitsteil 112 gekoppelt, das am proximalen Ende verteilte Spiegelelemente 114 aufweist, kann von diesen das radial eingespeiste Beleuchtungslicht nach distal umgelenkt und weitergeleitet werden. Die Weiterleitung kann dann unterschiedlich erfolgen, entweder durch das Material des Arbeitsteils 112 selbst oder durch entsprechende Lichtleitfasern oder dergleichen.

Alle drei Varianten von Fig. 8, 9 und 10 erlauben eine relative Verdrehbärkeit zwischen Arbeitsteil und Bedienteil, ohne dass die Einstellung einen Einfluss auf die Beleuchtung hat.

Die in Fig. 10 gezeigte Variante ist hier zwischen Arbeitsteil 112 und Bedienteil 116 dargestellt. Das in Fig. 10 dargestellte Bedienteil 116 kann aber auch ein Außenteil des Arbeitsteils 112 sein, wie das zuvor in der Variante von Fig. 1 bis 5 beschrieben wurde, d.h. die Drehbarkeit ist dann im Arbeitsteil selbst gegeben, nämlich einmal zwischen dem Innenteil mit den Spiegelelementen und das diesen umgebenden Außenteil mit den radial abstrahlenden LEDs.

## Patentansprüche

1. Videoendoskop, mit einem Arbeitsteil (12, 80, 92, 102, 112), wobei das Arbeitsteil einen Schaft (20, 82) aufweist, an dessen distalem Ende (22) ein Objektiv (24) und ein Bildsensor (32) angeordnet sind, und wobei das Arbeitsteil (12, 80, 92, 102, 112) ferner ein elektrisches Übertragungssystem (34) zum Übertragen der Bildinformation und zumindest einen Lichtleiter (26, 92, 102) zum Übertragen von Licht von proximal nach distal aufweist, und mit einem Bedienteil (14, 96, 116), wobei ein proximales Ende (48) des Arbeitsteils (12, 80, 92, 102, 112) mit einem distalen Ende (64) des Bedienteils (14, 96, 116) über eine Schnittstelle (16) lösbar verbindbar ist, wobei am Arbeitsteil (80) ein Schlauch angebracht ist, mit dem das Bedienteil (14) umhüllbar ist, **dadurch gekennzeichnet, dass** der Schlauch als steriler zusammengeraffter Schlauch (27, 58) ausgebildet ist, und dass das Arbeitsteil (12, 80, 92, 102, 112) als Einweginstrument ausgebildet ist.

2. Videoendoskop nach Anspruch 1, **dadurch gekennzeichnet, dass** die Schnittstelle (16) derart ausgebildet ist, dass die Bildinformation von dem Bildsensor (32) an das Bedienteil (14) übertragbar ist.

3. Videoendoskop nach Anspruch 1 oder 2, **dadurch gekennzeichnet, dass** die Schnittstelle (16) derart ausgebildet ist, dass Licht von dem Bedienteil (14, 96, 116) an das Arbeitsteil (12, 92, 112) übertragbar ist.

4. Videoendoskop nach einem der Ansprüche 1 bis 3, **dadurch gekennzeichnet, dass** das Arbeitsteil (12) ein Außenteil (18) aufweist, das als Schaft (20) ausgebildet ist.

5. Videoendoskop nach einem der Ansprüche 1 bis 4, **dadurch gekennzeichnet, dass** in dem Schaft (20) der zumindest eine Lichtleiter (26, 92, 102) angeordnet ist.

6. Videoendoskop nach einem der Ansprüche 1 bis 5, **dadurch gekennzeichnet, dass** das Arbeitsteil (12) ein Innenteil (30) aufweist, in dem der Bildsensor (32) und das elektrische Übertragungssystem (34) angeordnet sind.

7. Videoendoskop nach Anspruch 6 in kombination mit Anspruch 4, **dadurch gekennzeichnet, dass** das Außenteil (18) relativ zu dem Innenteil (30) drehbar ist.

8. Videoendoskop nach einem der Ansprüche 4 oder 7, **dadurch gekennzeichnet, dass** ein Mechanismus (42) zum Drehen des Außenteils (18) vorgesehen ist.

9. Videoendoskop nach einem der Ansprüche 1 bis 8, **dadurch gekennzeichnet, dass** der Bildsensor (32) als CMOS-Sensor ausgebildet ist.

10. Videoendoskop nach einem der Ansprüche 1 bis 8, **dadurch gekennzeichnet, dass** der Bildsensor (32) als CCD-Sensor ausgebildet ist.

11. Videoendoskop nach einem der Ansprüche 1 bis 10, **dadurch gekennzeichnet, dass** das elektrische Übertragungssystem (34) als zumindest eine durchgehende Platine (36) ausgebildet ist.

12. Videoendoskop nach Anspruch 11, **dadurch gekennzeichnet, dass** ein proximales Ende der Platine (36) als Stecker (38) ausgebildet ist.

13. Videoendoskop nach Anspruch 11 oder 12, **dadurch gekennzeichnet, dass** der Bildsensor (32) und die Platine (36) von einem zylinderförmigen Element (40) umschlossen sind.

14. Videoendoskop nach einem der Ansprüche 1 bis 13, **dadurch gekennzeichnet, dass** das Arbeitsteil (12) zumindest einen Kanal (84) aufweist.

15. Videoendoskop nach einem der Ansprüche 1 bis 14, **dadurch gekennzeichnet, dass** das proximale Ende (48) des Arbeitsteils (12) einen größeren Durchmesser aufweist als das distale Ende (22).

16. Videoendoskop nach einem der Ansprüche 1 bis 15, **dadurch gekennzeichnet, dass** in dem Arbeitsteil (102) LEDs (106) angeordnet sind.

17. Videoendoskop nach Anspruch 16, **dadurch gekennzeichnet, dass** LEDs (106) im Innenteil (18) angeordnet sind.

18. Videoendoskop nach einem der Ansprüche 1 bis 17, **dadurch gekennzeichnet, dass** an dem proximalen Ende (48) des Arbeitsteils (12) ein Lichteintritt (54) angeordnet ist.

19. Videoendoskop nach einem der Ansprüche 1 bis 18, **dadurch gekennzeichnet, dass** an dem distalen Ende (64) des Bedienteils (14) ein Lichtaustritt (74) angeordnet ist.

20. Videoendoskop nach Ansprüch 12, **dadurch gekennzeichnet, dass** an dem distalen Ende (64) des Bedienteils (14) eine Buchse (66) zur Aufnahme des Steckers (38) angeordnet ist.

21. Videoendoskop nach einem der Ansprüche 1 bis 20, **dadurch gekennzeichnet, dass** das Bedienteil (14) Bedienelemente (76, 77, 78, 79) aufweist.

22. Videoendoskop nach Ansprüch 18, **dadurch gekennzeichnet, dass** im distalen Bereich des Bedienteils (96, 116) umfänglich LEDs (98, 120) angeordnet sind, die den Lichteintritt des Arbeitsteils (92, 112) beleuchten.

23. Videoendoskop nach Anspruch 18, **dadurch gekennzeichnet, dass** im distalen Bereich des Bedienteils (96) umfänglich verteilt Lichtleitfasern (100) angeordnet sind, die den Lichteintritt des Arbeitsteils (92) beleuchten.

24. Videoendoskop nach einem der Ansprüche 18 bis 22, **dadurch gekennzeichnet, dass** im Bedienteil (116) umfänglich verteilt LEDs (120) angeordnet sind, die Licht radial in das Arbeitsteil (112) einkoppeln, welches über Spiegelelemente (114) nach distal umlenkbar ist.

## Claims

1. Video endoscope comprising a working part (12, 80, 92, 102, 112), wherein the working part has a shaft (20, 82), at the distal end (22) of which a lens (24) and an image sensor (32) are arranged, and wherein the working part (12, 80, 92, 102, 112) furthermore has an electrical transmission system (34) for transmitting the image information and at least one light guide (26, 92, 102) for transmitting light from proximal to distal, and comprising an operating part (14, 96, 116), wherein a proximal end (48) of the working part (12, 80, 92, 102, 112) can be releasably connected to a distal end (64) of the operating part (14, 96, 116) via an interface (16), wherein a hose is arranged at the working part (80), the operating part (14) can be enveloped by said hose, **characterized in that** the hose is formed as a sterile gathered-together hose (27, 58), and **in that** the working part (12, 80, 92, 102, 112) is formed as a one-way instrument.

2. Video endoscope of claim 1, **characterized in that** the interface (16) is formed in such a way that the image information can be transmitted from the image sensor (32) to the operating part (14).

3. Video endoscope of claim 1 or 2, **characterized in that** the interface (16) is formed in such a way that light can be transmitted from the operating part (14, 96, 116) to the working part (12, 92, 112).

4. Video endoscope of any one of claims 1 to 3, **characterized in that** the working part (12) comprises an outer part (18) formed as a shaft (20).

5. Video endoscope of any one of claims 1 to 4, **characterized in that** the at least one light guide (26, 92, 102) is arranged in the shaft (20).

6. Video endoscope of any one of claims 1 to 5, **characterized in that** the working part (12) comprises an inner part (30), in which the image sensor (32) and the electrical transmission system (34) are arranged.

7. Video endoscope of claim 6 in combination with claim 4, **characterized in that** the outer part (18) can be rotated relative to the inner part (30).

8. Video endoscope, of any one of claims 4 or 7, **characterized in that** a mechanism (42) for rotating the outer part (18) is provided.

9. Video endoscope of any one of claims 1 to 8, **characterized in that** the image sensor (32) is formed as a CMOS sensor.

10. Video endoscope of any one of claims 1 to 8, **characterized in that** the image sensor (32) is formed as a CCD sensor.

11. Video endoscope of any one of claims 1 to 10, **characterized in that** the electrical transmission system (34) is formed as at least one continuous circuit board (36).

12. Video endoscope of claim 11, **characterized in that** a proximal end of the circuit board (36) is formed as a plug (38).

13. Video endoscope of claim 11 or 12, **characterized in that** the image sensor (32) and the circuit board (36) are enclosed by a cylindrical element (40).

14. Video endoscope of any one of claims 1 to 13, **characterized in that** the working part (12) has at least one channel (84).

15. Video endoscope of any one of claims 1 to 14, **characterized in that** the proximal end (48) of the working part (12) has a larger diameter than the distal end (22).

16. Video endoscope of any one of claims 1 to 15, **characterized in that** LEDs (106) are arranged in the working part (102).

17. Video endoscope of claim 16, **characterized in that** LEDs (106) are arranged in the inner part (18).

18. Video endoscope of any one of claims 1 to 17, **characterized in that** a light entrance (54) is arranged at the proximal end (48) of the working part (12).

19. Video endoscope of any one of claims 1 to 18, **characterized in that** a light exit (74) is arranged at the distal end (64) of the operating part (14).

20. Video endoscope of claim 12, **characterized in that** a socket (66) for receiving the plug (38) is arranged at the distal end (64) of the operating part (14).

21. Video endoscope of any one of claims 1 to 20, **characterized in that** the operating part (14) has operating elements (76, 77, 78, 79).

22. Video endoscope of claim 18, **characterized in that** LEDs (98, 120) are arranged circumferentially in the distal region of the operating part (96, 116), said LEDs (98, 120) illuminating the light entrance of the working part (92, 112).

23. Video endoscope of claim 18, **characterized in that** optical fibres (100) are arranged in a manner distributed circumferentially in the distal region of the operating part (96), said optical fibres illuminating the light entrance of the working part (92).

24. Video endoscope of any one of claims 18 to 22, **characterized in that** LEDs (120) are arranged in a manner distributed circumferentially in the operating part (116), said LEDs coupling light radially into the working part (112), which can be deflected towards the distal end by means of mirror elements (114).

## Revendications

1. Vidéo-endoscope, avec une partie de travail (12, 80, 92, 102, 112), laquelle partie de travail présente une tige (20, 82) à l'extrémité distale (22) de laquelle sont disposés un objectif (24) et un capteur d'image (32), et laquelle partie de travail (12, 80, 92, 102, 112) présente également un système de transmission électrique (34) pour la transmission de l'information d'image et au moins un guide de lumière (26, 92, 102) pour la transmission de lumière de proximal à distal, et avec une partie de commande (14, 96, 116), une extrémité proximale (48) de la partie de travail (12, 80, 92, 102, 112) pouvant être reliée de manière détachable à une extrémité distale (64) de la partie de commande (14, 96, 116) via une interface (16), un tuyau étant monté sur la partie de travail (80), avec lequel la partie de commande (14) peut être enveloppée, **caractérisé en ce que** le tuyau est réalisé sous la forme d'un tuyau (27, 58) stérile replié sur lui-même, et que la partie de travail (12, 80, 92, 102, 112) est réalisée sous la forme d'un instrument à usage unique.

2. Vidéo-endoscope selon la revendication 1, **caractérisé en ce que** l'interface (16) est conçue de façon que l'information d'image du capteur d'image (32) puisse être transmise à la partie de commande (14).

3. Vidéo-endoscope selon la revendication 1 ou 2, **caractérisé en ce que** l'interface (16) est conçue de façon que la lumière puisse être transmise de la partie de commande (14, 96, 116) à la partie de travail (12, 92, 112).

4. Vidéo-endoscope selon une des revendications 1 à 3, **caractérisé en ce que** la partie de travail (12) présente une partie extérieure (18) qui est réalisée sous la forme d'une tige (20).

5. Vidéo-endoscope selon une des revendications 1 à 4, **caractérisé en ce que** l'au moins un guide de lumière (26, 92, 102) est disposé dans la tige (20).

6. Vidéo-endoscope selon une des revendications 1 à 5, **caractérisé en ce que** la partie de travail (12) présente une partie intérieure (30) dans laquelle le capteur d'image (32) et le système de transmission électrique (34) sont disposés.

7. Vidéo-endoscope selon la revendication 6 en combinaison avec la revendication 4, **caractérisé en ce que** la partie extérieure (18) peut tourner par rapport à la partie intérieure (30).

8. Vidéo-endoscope selon une des revendications 4 ou 7, **caractérisé en ce qu'**il est prévu un mécanisme (42) pour faire tourner la partie extérieure (18).

9. Vidéo-endoscope selon une des revendications 1 à 8, **caractérisé en ce que** le capteur d'image (32) est réalisé sous la forme d'un capteur CMOS.

10. Vidéo-endoscope selon une des revendications 1 à 8, **caractérisé en ce que** le capteur d'image (32) est réalisé sous la forme d'un capteur CCD.

11. Vidéo-endoscope selon une des revendications 1 à 10, **caractérisé en ce que** le système de transmission électrique (34) est réalisé sous la forme d'au moins une platine continue (36).

12. Vidéo-endoscope selon la revendication 11, **caractérisé en ce qu'**une extrémité proximale de la platine (36) est réalisée sous la forme d'un connecteur (38).

13. Vidéo-endoscope selon la revendication 11 ou 12, **caractérisé en ce que** le capteur d'image (32) et la platine (36) sont renfermés par un élément cylindrique (40).

14. Vidéo-endoscope selon une des revendications 1 à 13, **caractérisé en ce que** la partie de travail (12) présente au moins un canal (84).

15. Vidéo-endoscope selon une des revendications 1 à 14, **caractérisé en ce que** l'extrémité proximale (48) de la partie de travail (12) présente un plus grand diamètre que l'extrémité distale (22).

16. Vidéo-endoscope selon une des revendications 1 à 15, **caractérisé en ce que** des LED (106) sont disposées dans la partie de travail (102).

17. Vidéo-endoscope selon la revendication 16, **caractérisé en ce que** des LED (106) sont disposées dans la partie intérieure (18).

18. Vidéo-endoscope selon une des revendications 1 à 17, **caractérisé en ce qu'**une entrée de lumière (54) est disposée à l'extrémité proximale (48) de la partie de travail (12).

19. Vidéo-endoscope selon une des revendications 1 à 18, **caractérisé en ce qu'**une sortie de lumière (74) est disposée à l'extrémité distale (64) de la partie de commande (14).

20. Vidéo-endoscope selon la revendication 12, **caractérisé en ce qu'**une douille (66) pour recevoir le connecteur (38) est disposée à l'extrémité distale (64) de la partie de commande (14).

21. Vidéo-endoscope selon une des revendications 1 à 20, **caractérisé en ce que** la partie de commande (14) présente des éléments de commande (76, 77, 78, 79).

22. Vidéo-endoscope selon la revendication 18, **caractérisé en ce que** des LED (98, 120) qui éclairent l'entrée de lumière de la partie de travail (92, 112) sont disposées périphériquement dans la zone distale de la partie de commande (96, 116).

23. Vidéo-endoscope selon la revendication 18, **caractérisé en ce que** des fibres optiques (100) qui éclairent l'entrée de lumière de la partie de travail (92) sont réparties périphériquement dans la zone distale de la partie de commande (96).

24. Vidéo-endoscope selon une des revendications 18 à 22, **caractérisé en ce que** des LED (120) qui sont réparties périphériquement dans la partie de commande (116) injectent de la lumière radialement dans la partie de travail (112), laquelle lumière peut être déviée vers distal au moyen d'éléments de miroir (114).
